# EUROPÄISCHE PATENTSCHRIFT

(11) **EP 0 566 974 B1**
(45) Veröffentlichungstag und Bekanntmachung des Hinweises auf die Patenterteilung: **27.09.1995**
(21) Anmeldenummer: 93106036.2
(22) Anmeldetag: 14.04.1993
(51) Int. Cl.: C07C 53/21, C07C 51/487

(54) **Reinigung von fluorierten Carbonsäuren**
Purification of fluorinated carboxylic acids
Purification d'acides carboxyliques fluorés

(30) Priorität: 22.04.1992 DE 4213154
(43) Veröffentlichungstag der Anmeldung: 27.10.1993
(73) Patentinhaber: HOECHST AKTIENGESELLSCHAFT, 65926 Frankfurt am Main (DE)
(72) Erfinder: Mayer, Ludwig, Dr., W-8263 Burghausen (DE); Löhr, Gernot, Dr., W-8269 Burgkirchen (DE)

(56) Entgegenhaltungen:
- DE-A- 2 104 828
- US-A- 3 833 626

## Beschreibung

Für die Polymerisation fluorierter Monomerer in wäßriger Dispersion werden fluorierte Emulgatoren eingesetzt, da diese keine telogene Aktivität aufweisen. Hauptsächlich werden gutlösliche Salze von Perfluorcarbonsäuren, insbesondere das Ammoniumsalz der Perfluoroctansäure, verwendet. Für diesen Zweck müssen die zugrundeliegenden Säuren eine hohe Reinheit aufweisen, da Verunreinigungen das Anspringen der Polymerisation be- oder gar verhindern können, Störungen im Polymerisationsablauf, beispielsweise durch Kettenübertragung, auftreten können oder ein Abbruch der Polymerisation erfolgen kann.

Häufig werden derartige Störungen auch beim Einsatz von Handelsprodukten festgestellt, auch wenn in diesen keine Verunreinigungen ersichtlich sind. Es empfiehlt sich deshalb, für die Verwendung als Emulgatoren die zugrundeliegenden fluorierten Säuren einer speziellen Reinigung zu unterziehen.

Aus der veröffentlichten japanischen Patentanmeldung 89/117840 ist es bekannt, n-Perfluoroctansäure aus einer Mischung mit Isoperfluoroctansäure durch Kristallisieren aus wäßrigem Chloroform in reiner Form zu gewinnen. Chloroform ist jedoch toxisch und gilt als kanzerogen, so daß eine industrielle Realisierung dieses Verfahrens einen außerordentlichen Aufwand mit sich brächte.

Es wurde nun gefunden, daß fluorierte Carbonsäuren in die genannte erforderliche Reinheit überführt werden können, wenn man das Ausgangsmaterial zunächst - falls erforderlich - weitgehend entwässert, das wasserarme Produkt mit Oxidationsmitteln behandelt und das Reinprodukt abtrennt.

Der für die Behandlung mit dem Oxidationsmittel erforderliche niedrige Wassergehalt kann von der Art und Menge der Verunreinigungen und der Wahl des Oxidationsmittels abhängen und wird zweckmäßig durch einfache Vorversuche ermittelt. Üblicherweise stören Wassermengen von unter 2 Gew.-% nicht.

Als Ausgangsmaterial können Produkte dienen, die aus 1-Iodperfluoralkanen gewonnen wurden, beispielsweise nach dem Verfahren der US-Patentschrift 4 400 325. Weiterhin sind Produkte geeignet, die aus Abwässern mit Hilfe basischer Anionenaustauscher erhalten wurden, beispielsweise Produkte nach dem Verfahren der US-Patentschrift 4 282 162. Wenn man das Verfahren für handelsübliche "Reinmaterialien" einsetzt, kann - je nach deren Wassergehalt - auf den Entwässerungsschritt verzichtet werden.

Die Entwässerung des Ausgangsmaterials kann in an sich bekannter Weise erfolgen, also durch den Zusatz wasserentziehender Mittel oder mit physikalischen Methoden. Als wasserentziehende Mittel kommen grundsätzlich Salze wie Natriumsulfat oder Calciumchlorid in Betracht, wobei deren Einsatz jedoch das weitere Verfahren, also die Oxidation oder die Abtrennung des Reinproduktes, beeinträchtigen kann. Vorteilhaft wählt man deshalb Zusätze wie Phosphorpentoxid, insbesondere aber konzentrierte Schwefelsäure, Schwefeltrioxid oder deren Mischungen ("Oleum").

Unter den physikalischen Entwässerungsmethoden ist die Destillation bevorzugt, insbesondere dann, wenn auch die Abtrennung des Reinprodukts durch Destillation erfolgen soll. Bei dieser Ausgestaltung der Erfindung wird also in einem ersten Schritt der störende Wassergehalt reduziert und - zweckmäßig in der gleichen Apparatur - nach der Behandlung mit dem Oxidationsmittel das Reinprodukt abdestilliert.

Die zu reinigenden fluorierten Carbonsäuren sind zwar relativ starke Säuren, es empfiehlt sich jedoch der Zusatz noch stärkerer Säuren, wie zum Beispiel konzentrierter Schwefelsäure, da die Oxidationsmittel ihre beste Wirkung im sauren Bereich zeigen.

Zweckmäßig erfolgt die Behandlung mit dem Oxidationsmittel unter Erwärmung auf eine Temperatur, bei der eine gute Durchmischung gewährleistet ist. Vorteilhaft arbeitet man in einer homogenen, flüssigen Phase, beispielsweise im Bereich von etwa 60 °C bis zum Siedepunkt des Gemisches.

Art und Menge des Oxidationsmittels wählt man zweckmäßig anhand einfacher Vorversuche, insbesondere dann, wenn - wie üblich - Art und Umfang der Verunreinigungen nicht oder nicht genau genug bekannt sind. Als Erfolgskriterium kann ein Polymerisationsansatz von Tetrafluorethylen (TFE) mit Ammoniumpersulfat als Initiator unter üblichen Bedingungen dienen.

Als Oxidationsmittel dienen alle Redox-Systeme mit Normalpotentialen größer +1,30 V, bevorzugt Dichromate und Permanganate und vor allem Persulfate wie Natrium-, Kalium- oder Ammoniumperoxodisulfat.

Die Abtrennung der gereinigten Fluoralkansäure kann durch Kristallisation erfolgen. n-Perfluoroctansäure-(1) schmilzt bei 54 bis 55 °C. Eine Kristallisation kann in diesem Falle also durch entsprechende - zweckmäßig langsame - Abkühlung, gegebenenfalls unter Zusatz eines Lösemittels wie Wasser, das die unerwünschten Bestandteile der Reaktionsmischung in Lösung hält, erfolgen.

Vorteilhaft ist die Isolierung des gereinigten Produktes durch Destillation, gegebenenfalls unter vermindertem Druck. n-Perfluoroctansäure-(1) siedet bei einem Druck von 5,3 kPa bei 113 °C. Es ist deshalb eine Abtrennung in der gewünschten hohen Reinheit durch fraktionierte Destillation im Wasserstrahlvakuum möglich.

Die erfindungsgemäß gereinigten Fluorcarbonsäuren eignen sich in Form ihrer leichtlöslichen Salze, vorzugsweise der Ammoniumsalze, als Emulgatoren für die Polymerisation fluorierter Monomerer in wäßriger Dispersion. Die Erfindung erlaubt somit die Rückgewinnung dieser wertvollen Substanzen in hohen Ausbeuten auch aus verunreinigten und stark verdünnten Medien, beispielsweise aus Produktionsabwässern.

Bevorzugte Ausgestaltungen der Erfindung sind aus den folgenden Beispielen ersichtlich. Prozentangaben beziehen sich auf das Gewicht.

### Beispiel 1

In einem 6-l-Rührkolben, versehen mit Bodenablaßhahn und Heizpilz, werden 3000 g Salzbrei mit folgender Zusammensetzung eingesetzt:
- circa 40 % Natriumsalz der Perfluoroctansäure (PFOS)
- circa 20 % Natriumsulfat
- unter 5 % Natriumhydroxid
- circa 40 % Wasser
Unter langsamem Rühren des Salzbreis bei 20 °C tropft man in circa 20 Minuten 300 ml konzentrierte Schwefelsäure zu.

Der etwa 50 bis 60 °C heiße Kolbeninhalt wird auf 65 °C erwärmt, in einen Scheidetrichter gegeben und die PFOS-Unterphase (1303 g, Wassergehalt: 9 %) abgetrennt. 200 g dieser wasserhaltigen Roh-PFOS werden in einem 500-ml-Rührkolben mit 80 g Oleum (SO₃-Gehalt: 20 %) versetzt, auf circa 60 °C erwärmt und unter Rühren in kleinen Portionen 14 g Ammoniumperoxidisulfat (APS) zugegeben. Dabei tritt starke Erwärmung und Aufschäumen auf. Dieses Gemisch wird unter langsamem Rühren 7 Stunden bei 100 bis 110 °C gehalten.

Nach Aufsetzen eines kurzen Kolonnenteils und eines auf 60 °C beheizten Kolonnenkopfes wird nun nach Abnahme von circa 10 % Vorlauf die PFOS im Wasserstrahlvakuum überdestilliert (Menge des Hauptlaufs: 115 g). Die PFOS ist farblos und verändert sich auch über Monate nicht. Bei der TFE-Polymerisation beziehungsweise -Copolymerisation können im Vergleich zu hochreiner Handelsware keine Unterschiede bezüglich TFE-Aufnahme, Molekulargewicht des resultierenden Polymeren, Initiatorverbrauch oder ähnliches festgestellt werden.

### Beispiel 2

200 g einer braun verfärbten, nach dem Verfahren der US-Patentschrift 4 282 162 rückgewonnenen, trockenen PFOS werden in einem 500-ml-Rührkolben mit 80 g konzentrierter Schwefelsäure versetzt, auf circa 60 °C erwärmt und in kleinen Portionen 14 g APS zugegeben. Dieses Gemisch wird unter langsamem Rühren 7 Stunden bei 100 bis 110 °C gehalten. Nach Aufsetzen eines kurzen Kolonnenteils und eines auf 60 °C beheizten Kolonnenkopfes wird nach Abnahme von circa 10 % Vorlauf die PFOS im Wasserstrahlvakuum überdestilliert (Menge des Hauptlaufs: 159 g). Die PFOS ist farblos und verändert sich nicht mehr.

Diese mit APS nachbehandelte, rückgewonnene PFOS besitzt "polymerisation grade", das heißt, TFE-Polymerisation und -Copolymerisation können innerhalb der üblichen statistisch kontrollierten Prozeßparameter durchgeführt werden.

### Beispiel 3

102 g eines Natriumperfluoroctanat-haltigen Salzes folgender Zusammensetzung:
- circa 53 % Natriumsalz der PFOS
- circa 36 % Natriumsulfat
- circa 6,5 % Natriumhydroxid
- 4,5 % Wasser
werden in einem 500-ml-Rührkolben mit 40 g konzentrierter Schwefelsäure versetzt und auf circa 60 °C erwärmt. Unter Rühren werden 7 g Natriumdichromat zugegeben. Dieses Gemisch wird 5,5 Stunden unter langsamem Rühren bei 100 bis 120 °C gehalten.

Nach Aufsetzen eines kurzen Kolonnenteils und eines auf 60 °C beheizten Kolonnenkopfes wird nun im Wasserstrahlvakuum die PFOS überdestilliert. Man erhält 41 g farblose PFOS, die sich nicht mehr verändert.

### Vergleichsbeispiel

200 g braun verfärbte Roh-PFOS mit 9 % Wassergehalt werden in einem 500-ml-Kolben mit 40 g konzentrierter Schwefelsäure versetzt und über ein kurzes Kolonnenteil mit einem auf 60 °C beheizten Kolonnenkopf im Wasserstrahlvakuum destilliert. Nach Abnahme von circa 10 % Vorlauf werden 142 g PFOS als Hauptlauf abdestilliert. Die PFOS ist farblos, verfärbt sich aber in circa 7 Tagen dunkelbraun.

Führt man mit dieser PFOS TFE-Polymerisationsversuche durch, so beobachtet man im Vergleich zu der PFOS aus Beispiel 1 oder 2 erhöhte Laufzeiten und die Bildung niedrigerer Molekulargewichte.

## Patentansprüche

1. Verfahren zur Reinigung von fluorierten Carbonsäuren, dadurch gekennzeichnet, daß man wasserarme Ausgangsmaterialien mit Oxidationsmitteln behandelt und das gereinigte Produkt abtrennt.

2. Verfahren nach Anspruch 1, dadurch gekennzeichnet, daß man ein Ausgangsmaterial mit einem Wassergehalt unter 2 Gew.-% einsetzt.

3. Verfahren nach Anspruch 1, dadurch gekennzeichnet, daß man ein Ausgangsmaterial mit unerwünscht hohem Wassergehalt mit chemischen oder physikalischen Mitteln behandelt und das wasserarme Produkt mit einem Oxidationsmittel versetzt.

4. Verfahren nach Anspruch 3, dadurch gekennzeichnet, daß die chemische Wasserentziehung mit Schwefelsäure und/oder Schwefeltrioxid erfolgt.

5. Verfahren nach Anspruch 3, dadurch gekennzeichnet, daß die Wasserabtrennung destillativ erfolgt.

6. Verfahren nach einem oder mehreren der vorhergehenden Ansprüche, dadurch gekennzeichnet, daß die Behandlung bei einer Temperatur erfolgt, die gute Durchmischung des Reaktionsmediums gewährleistet.

7. Verfahren nach einem oder mehreren der vorhergehenden Ansprüche, dadurch gekennzeichnet, daß die Behandlung bei einer Temperatur von 60 °C bis zum Siedepunkt des Reaktionsgemisches erfolgt.

8. Verfahren nach einem oder mehreren der vorhergehenden Ansprüche, dadurch gekennzeichnet, daß als Oxidationsmittel ein Redox-System mit einem Normalpotential größer +1,30 V eingesetzt wird.

9. Verfahren nach einem oder mehreren der vorhergehenden Ansprüche, dadurch gekennzeichnet, daß als Oxidationsmittel ein Persulfat, Permanganat oder Dichromat eingesetzt wird.

10. Verfahren nach einem oder mehreren der vorhergehenden Ansprüche, dadurch gekennzeichnet, daß die Abtrennung des Reinproduktes durch Destillation erfolgt.

## Claims

1. A process for the purification of fluorinated carboxylic acids, which comprises treating low-water starting materials with oxidants and isolating the purified product.

2. The process as claimed in claim 1, wherein a starting material is used having a water content below 2 % by weight.

3. The process as claimed in claim 1, wherein a starting material having undesirably high water content is treated by chemical or physical means and an oxidant is added to the low-water product.

4. The process as claimed in claim 3, wherein the chemical dehydration is carried out using sulfuric acid and/or sulfur trioxide.

5. The process as claimed in claim 3, wherein the water removal is carried out by distillation.

6. The process as claimed in one or more of the preceding claims, wherein the treatment is carried out at a temperature which ensures thorough mixing of the reaction medium.

7. The process as claimed in one or more of the preceding claims, wherein the treatment is carried out at a temperature from 60 °C to the boiling point of the reaction mixture.

8. The process as claimed in one or more of the preceding claims wherein the oxidant used is a redox system having a standard electrode potential greater than +1.30 V.

9. The process as claimed in one or more of the preceding claims, wherein the oxidant used is a persulfate, permanganate or dichromate.

10. The process as claimed in one or more of the preceding claims, wherein the isolation of the pure product is carried out by distillation.

## Revendications

1. Procédé de purification d'acides carboxyliques fluorés, caractérisé en ce qu'on traite des matériaux de départ appauvris en eau avec des agents d'oxydation et on sépare le produit purifié.

2. Procédé selon la revendication 1, caractérisé en ce qu'on utilise un matériau de départ ayant une teneur en eau inférieure à 2 % en poids.

3. Procédé selon la revendication 1, caractérisé en ce qu'on traite un matériau de départ ayant une teneur en eau élevée non désirée par des moyens chimiques ou physiques et on additionne un agent d'oxydation au produit appauvri en eau.

4. Procédé selon la revendication 3, caractérisé en ce que la déshydratation chimique se fait avec de l'acide sulfurique et/ou du trioxyde de soufre.

5. Procédé selon la revendication 3, caractérisé en ce que l'élimination d'eau se fait par distillation.

6. Procédé selon une ou plusieurs des revendications précédentes, caractérisé en ce que le traitement se fait à une température garantissant un bon mélange du milieu réactionnel.

7. Procédé selon une ou plusieurs des revendications précédentes, caractérisé en ce que le traitement se fait à une température comprise entre 60°C et le point d'ébullition du mélange réactionnel.

8. Procédé selon une ou plusieurs des revendications précédentes, caractérisé en ce qu'on utilise comme agent d'oxydation un système Redox ayant un potentiel de référence supérieur à +1,30 V.

9. Procédé selon une ou plusieurs des revendications précédentes, caractérisé en ce qu'on utilise, comme agent d'oxydation, un persulfate, un permanganate ou un dichromate.

10. Procédé selon l'une ou plusieurs des revendications précédentes, caractérisé en ce que la séparation du produit pur se fait par distillation.
